# EUROPEAN PATENT SPECIFICATION

(11) **EP 2 637 614 B1**
(45) Date of publication and mention of the grant of the patent: **20.02.2019**
(21) Application number: 11794022.1
(22) Date of filing: 10.11.2011
(51) Int. Cl.: A61F 5/445, A61F 5/448

(54) **CONNECTING MEMBER AND CONNECTOR FOR AN OSTOMY DEVICE**
VERBINDUNGSELEMENT UND VERBINDUNG FÜR EINE OSTOMIEVORRICHTUNG
ELÉMENT CONNECTEUR ET CONNECTEUR POUR UN DISPOSITIF D'OSTOMIE

(30) Priority: 10.11.2010 FR 1004419
(43) Date of publication of application: 18.09.2013
(73) Proprietor: B. Braun Medical Sas, 92210 Saint-Cloud (FR)
(72) Inventor: HOGARD, Aurélien, F-40390 Saint-Martin-de-Seignanx (FR)
(74) Representative: August Debouzy
(86) International application number: PCT/EP2011/005677
(87) International publication number: WO 2012/062472

(56) References cited:
- EP-A1- 0 799 608
- FR-A1- 2 913 593
- GB-A- 2 115 288
- US-A- 4 923 452

## Description

The present invention concerns a connecting member for an ostomy device as well as a connector for an ostomy device Document GB 2 115 288 A is regarded as the closest prior art.

An ostomy device is in general formed of two elements: a support intended to be affixed to the skin of a patient and a pouch forming a receptacle. These two elements must be joined in a secure and tight manner through two connecting members placed on the respective elements.

A connector for an ostomy device has two major functions, firstly to ensure that the weight of the device is supported, particularly when it contains evacuated matter, and secondly to prevent the passage of liquids and gas (unpleasant odours) to the outside of the device.

There are numerous types of connectors for ostomy devices, pertaining to two main categories, mechanical connectors and glued connectors. The invention relates to a connector of the mechanical type.

The support is usually glued on the patient's skin. For the patient's comfort, as well as for the good mounting of the pouch, it is necessary for the support to fit the patient's contours. The connecting member mounted on the support often prevents a good fit of the support, since the member is fairly rigid.

The object of the invention is therefore to make available to users an ostomy device offering greater comfort and fitting the patient's body better.

This problem is solved, according to the present invention, by means of a connecting member of an ostomy device which is mounted on a support intended to be affixed to the skin of a patient and which surrounds an opening in the support. The member according to the invention is characterised in that it comprises a first piece consisting of a flexible material and with two essentially parallel faces, the first of which is affixed to the support. The member further comprises at least one second piece consisting of a rigid material. This second piece is affixed onto the second face of the first piece and extends at least partially beyond the external contours of the first piece.

The connecting member according to the invention ensures a good fit between the support and the patient's skin. The first flexible piece does not prevent the easy deformation of the support in order to fit the contours of the patient. The second piece(s), which extend(s) slightly beyond the contours of said first piece(s), allow(s) a corresponding connecting element, such as for example a lip of a second connecting member, to be locked in behind the rim formed by the second piece(s). The second piece(s) are preferably relatively thin so that they are flexible despite being made from rigid material.

The connecting member may in particular comprise one or more second pieces, or, in other words, the second piece may be a single piece or consist of several pieces. A single second piece makes it simpler to manufacture said connecting member. Several second pieces increase the flexibility and fit of the connecting member.

Optionally, the connecting member may comprise a third piece consisting of rigid material and being arranged between the first piece and the support. The third piece shall extend at least partially beyond the external contours of the first piece so that it can serve as a dead stop when the pouch is mounted. Furthermore, the third piece ensures a good fit of the pouch on the first piece of the connecting member and avoids that the pouch may slip off the connecting member toward the support if the support is strongly deformed during the use.

The first piece of the connecting member according to the invention may in particular be ring-shaped. It may have two essentially parallel faces, one of which may be affixed to the support, or the third piece, respectively. The second face may be used to affix the second, rigid piece onto the first piece.

The second piece may also be essentially ring-shaped, with an external radius slightly larger than the external radius of the first piece, thus extending slightly beyond the contours of the first piece. The second piece may alternatively have sections with an external radius equal to or less than the radius of the first piece. Thus, the second piece extends only partially beyond the contours of the first piece. The second piece may be formed of a single piece or of two or more separate pieces.

The thickness of the first piece between its two faces is sufficient to allow a retaining element, such as for example a lip of a second connecting member to lock behind the rim formed by the second piece, extending beyond the contours of the first piece. The second piece is preferably much thinner than the first piece, in order to ensure a good fit and sufficient flexibility of the assembly despite the rigidity of the second piece.

For a secure connection, the second piece only has to extend beyond the external contours of the first piece by less than 1 mm and in particular by less than 0.5 mm.

The optional third piece is preferably also ring shaped, but any other shape, as for example square, rectangular or oval, may be used as well to achieve its function as dead stop. The third piece extends - in contrast to the second piece - not only slightly beyond the contours of the first piece. In order to maintain the flexibility of the support, the third piece is preferably only affixed to the support along its inner circumference. The remaining surface of the third piece towards its outer circumference is preferably not affixed to the support.

The first piece of the connecting member may preferably be made from a TPE (Thermoplastic Elastomer), such as for example styrene butadiene styrene (SBS), styrene ethylene butadiene styrene (SEBS), or ethyl vinyl acetate (EVA), or from a TPU (Thermoplastic polyurethane), or a silicone, or even a rubber. It preferably has a hardness between 0shA and 40shA, more particularly between 0shA and 20shA, and in particular 3shA.

The second piece may be made from polypropylene. The second piece preferably has a hardness between 60shD and 90shD, more particularly between 70shD and 80shD, and in particular 76shD.

The third piece may be made from TPE (Thermoplastic Elastomer), such as for example ethyl vinyl acetate (EVA), with a hardness between 60shA and 100shA, more particularly between 80shA and 100 shA, and in particular 95shA. The third piece may alternatively be made from polypropylene or low density polyethylene, and preferably from the same material as the second piece. It then has a hardness between 60shD and 90shD, more particularly between 70shD and 80shD, and in particular 76shD.

The connecting member may comprise optional spacer elements consisting of rigid material which are arranged between the second and third pieces. Such spacer elements are in particular useful if the hardness of the first piece is very low. The spacer elements limit the squeezing of the first piece when the pouch is mounted on the connecting member.

The spacer elements may be integral with the second and/or third piece(s) to facilitate the manufacturing and assembly of the pieces. If the spacer elements are integral with the second and third pieces they also limit the deformation of the first piece when the pouch is removed.

The spacer elements preferably have the form of small columns or thin walls which are spaced apart from each other to maintain the flexibility of the assembly despite the rigid material of the spacer elements.

The problem to be solved by the invention is also resolved by a connector for an ostomy device of the type comprising a first connecting member which is mounted on a support intended to be affixed to the skin of a user and of a second connecting member mounted on a pouch forming a receptacle. The first member of this connection surrounds an opening in the support and the second member surrounds an opening in the pouch. The first member has an external contour in a plane essentially parallel to the plane of the opening in the support, and the internal contour of the second member in a plane essentially parallel to the opening in the pouch is
essentially complementary but slightly larger than the external contour of the first member. The second member comprises on its internal side an elastically deforming lip, the free end of which is oriented towards the pouch. This lip reduces the internal dimensions of the second member to slightly less than the external dimensions of the first member. The first member of this connection has the characteristics of the connecting member described above.

The lip of said second connecting member may preferably be made from low adensity polyethylene. It preferably has a hardness between 20shD and 100shD, more particularly between 40shD and 60shD, and in particular 50shD.

Different embodiments of the invention are explained in greater detail in the following, with reference to the attached drawings:
- **Figure 1**: represents a first embodiment of a connecting member of an ostomy device in plan view;
- **Figure 2**: represents the connecting member from figure 1 in cross section along line A-A;
- **Figure 5**: represents a second embodiment of a connecting member of an ostomy device in plan view;

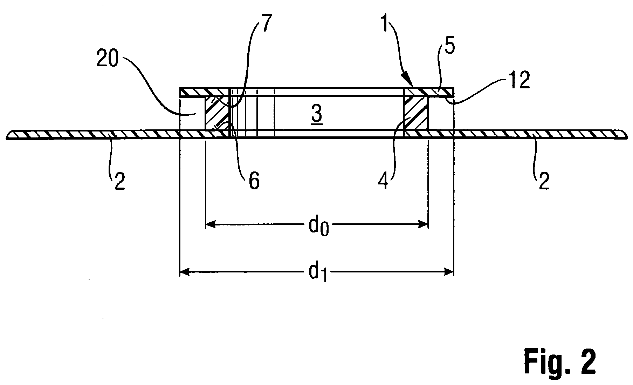

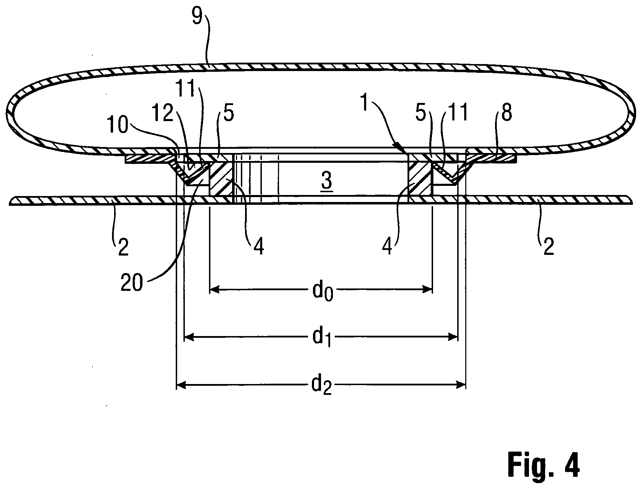

- **Figure 4**: represents an embodiment of a connector for an ostomy device with the connecting member from figure 2 in cross section;

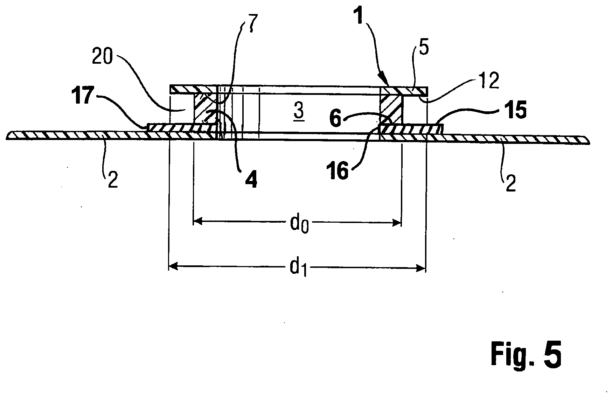

- **Figure 5**: represents a third embodiment of a connecting member in cross section;

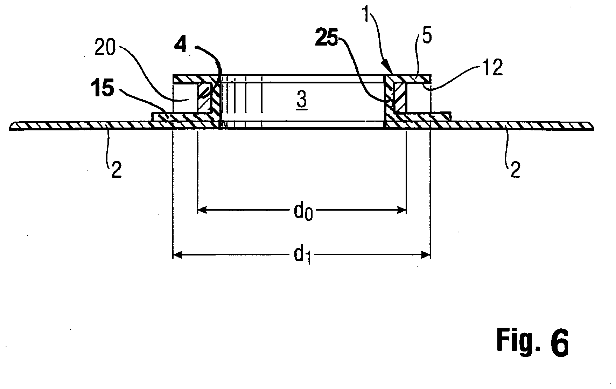

- **Figure 6**: represents a fourth embodiment of a connecting member in cross section.

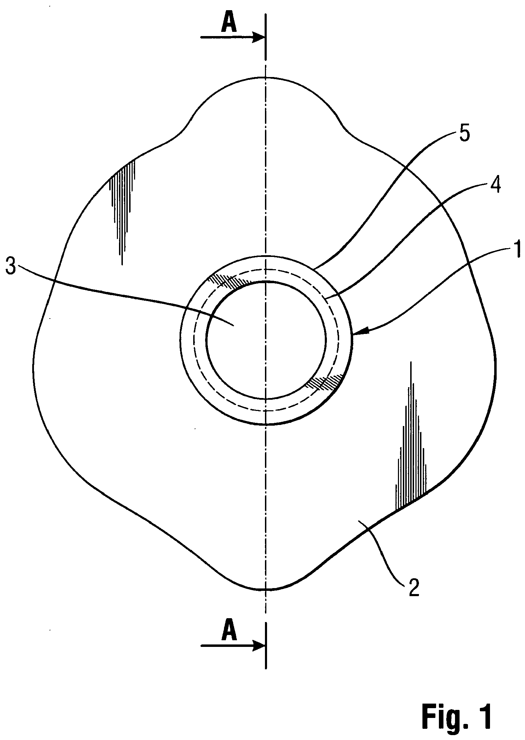

A first embodiment of the connecting member of an ostomy device according to the invention is shown in plan view in **figure 1**. The connecting member **1** is ring-shaped. It is mounted on a support **2** intended to be affixed to the skin of a patient. The connecting member **1** surrounds an opening **3** in the support **2.**

The member **1** comprises a first piece **4** made from styrene ethylene butadiene styrene (SEBS) with a hardness of 3shA. Said first piece **4** has two parallel faces of which one is affixed to the support **2.** The external contours of the first piece **4** are represented by a dotted line.

A second piece **5** made from polypropylene with a hardness of 76shD is affixed to the second face of said first piece **4.** The second piece **5** extends beyond the external contours of the first piece **4** along its entire circumference by around 0.5 mm.

**Figure 2** represents the connecting member from figure 1 in cross section along line **A-A.** The connecting member **1** is mounted on the support **2** and surrounds the opening **3** in the support **2.** One face **6** of the first piece **4** of the connecting member **1** is affixed to the support **2.**

A second piece **5** is affixed on the other face **7** of said first piece **4.** The second piece **5** extends beyond the external contours of the first piece **4** and thus forms a groove **20** to accommodate, for example, the lip of a complementary connecting member.

The support **2** is formed of a flexible material to better fit the contours of the patient. The first piece **4** of the connecting member **1** is also formed of a flexible material, in this case styrene ethylene butadiene styrene (SEBS), in order to fit the contours of the patient and in order not to reduce the flexibility of the support **2** too much.

The second piece **5** of the connecting member is a ring washer formed of rigid material, in this case polypropylene. The external diameter **d₁** of the washer is slightly greater than the external diameter **d₀** of the first piece **4.** However, the thickness of the second piece **5** is less than that of the first piece **4.** The second piece **5** thus forms a rim **12** behind which the connecting means of a second complementary connecting member may lock.

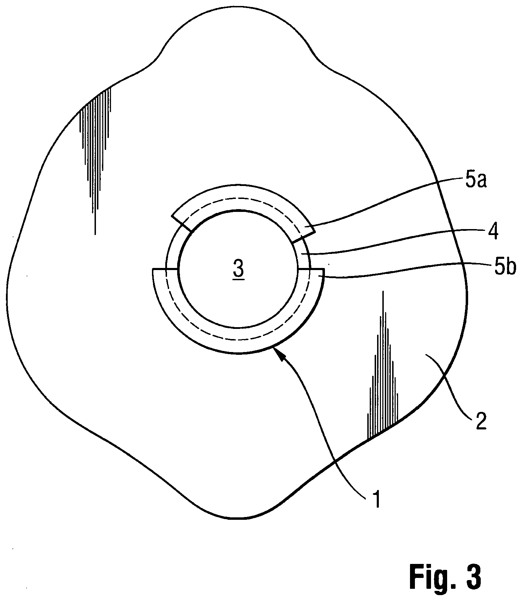

**Figure 3** shows a second embodiment of a connecting member **1** of an ostomy device shown in plan view. The member **1** according to this embodiment comprises a first ring-shaped piece **4** identical to that of figure 1, but two second pieces **5a** and **5b** that only partially exceed the circumference of said first piece **4.**

**Figure 4** represents an embodiment of a connector for an ostomy device in cross section. The connector is of the type comprising a first connecting member **1** mounted on a support **2** intended to be affixed to the skin of a patient and a second connecting member **8** mounted on a pouch **9** forming a receptacle. The first member **1** surrounds an opening **3** in the support **2** and the second member **8** surrounds an opening **3** in the pouch **9.**

The first member **1** in this embodiment is identical to the connecting member of figure 1. It is circular in shape and has an external diameter **d₁**. The second member **8** is also circular and has **an** internal diameter **d₂** slightly larger than the external diameter **d₁** of the first member **1.**

The second member **8** has on its internal side **10** an elastically deforming ring-shaped lip **11,** the free end of which is oriented towards the pouch **3,** that is, towards the top in the representation in **figure 4****.** This lip **11** reduces the internal diameter of the second member **8** to slightly less than the external diameter **d₁** of the first connecting member **1.** The second member **8** including the lip **11** is formed of high density polyethylene with a hardness of 50shD.

The pouch **9** may thus be attached to the support **2** by means of the two connecting members **1, 8.** When the two connecting members are connected, the lip **11** of the second connecting member **8** deforms elastically to lock behind the rim **12** of the second piece **5** of the first connecting member **1.** Since the second piece **5** is formed of a rigid material, the lip **11** is held securely behind its edge **12** extending beyond the first piece **5.** At the same time, the lip **11** of the second connecting member **8** forms a joint against the first piece **4** of the first connecting member **1.** Said first piece **4** is relatively flexible and may easily conform to the shape of the lip **11** to form a tight joint, the more so as the pressure in the pouch **9** increases.

**Figure 5** represents a connecting member with an optional third piece **15** arranged between the first piece **4** and the support **2.** The third piece **15** is made from rigid material, in the present case from ethyl vinyl acetate with a hardness of 95shA. It extends beyond the external contours of the first piece **4** for 10 mm to form a dead stop for the corresponding connecting member mounted on the pouch. The third piece **15** is relatively thin, in the present case 0,8 mm, to be flexible despite being made from rigid material. The third piece **15** is fixed to the support **2** only along its inner circumference **16,** i.e. essentially in the region where the first piece **4** is affixed to its opposite side. The remaining surface towards the outer circumference **17** of the third piece **15** is not affixed to the support **2** so that the support **2** keeps a maximum of its flexibility. The third piece **15** keeps its shape when the support **2** is deformed to adapt to the contour of a patient. The third piece **15** can hence still fulfil its function as dead stop for the matching connecting member of a pouch and avoids that the connecting member of the pouch slips off the first piece **4** towards the support **2** if the support **2** is strongly deformed during the use.

**Figure 6** shows another embodiment of the connecting member comprising a third piece **15** and spacer elements **25** between the third piece **15** and the second piece **5.** The spacer elements are integral with the second and third pieces **5, 15** and hence made from rigid material. They avoid that the first element **4** made from flexible material with a very low hardness is excessively squeezed when the connecting member of a pouch is mounted. Furthermore, the spacer elements **25** keep the assembly stable when the connecting element of a pouch is removed and avoids the rupture of the connecting element by excessive stretching of the flexible first piece **4.** The spacer elements **25** have the shape of small columns and are spaced so that the whole assembly is still quite flexible despite the rigid material of the spacer elements **25.**

## Claims

1. Connecting member (1) of an ostomy device,
the member (1) being mounted on a support (2) intended to be affixed to the skin of a patient
and surrounding an opening (3) in said support (2)
the member (1) comprising
a first piece (4) consisting of flexible material and with two essentially parallel faces (6, 7), the first of which (6) is affixed to the support (2),
and at least one second piece (5) consisting of rigid material,
being affixed to the second face (7) of the first piece (4) and extending at least partially beyond the external contours of the first piece (4),
**characterized by**
the thickness of the second piece (5) being less than the thickness of the first piece (4).

2. Connecting member (1) according to claim 1, **characterised in that** the member (1) comprises a third piece (15) consisting of rigid material and being arranged between the first piece (4) and the support (2), the third piece (15) extending at least partially beyond the external contours of the first piece.

3. Connecting member (1) according to claim 1 or 2, **characterised in that** the first piece (4) is ring-shaped.

4. Connecting member (1) according to claim 3, **characterised in that** the second piece (5) and/or the third piece (15) are essentially ring-shaped with an external radius at least in part slightly larger than the radius of the first piece (4).

5. Connecting member (1) according to one of claims 2 to 4, **characterised in that** the third piece (15) is affixed to the support along its inner circumference (16), the outer circumference (17) being not affixed to the support (2).

6. Connecting member (1) according to one of claims 1 to 5, **characterised in that** the first piece (4) is made from a TPE (Thermoplastic Elastomer), particularly in styrene butadiene styrene (SBS), styrene ethylene butadiene styrene (SEBS), or ethyl vinyl acetate (EVA), or from a TPU (Thermoplastic polyurethane), from a silicone, or from a rubber.

7. Connecting member (1) according to one of claims 1 to 6, **characterised in that** the second piece (5) is made of polypropylene.

8. Connecting member (1) according to one of claims 2 to 7, **characterised in that** the third piece (15) is made from a TPE (Thermoplastic Elastomer) particularly in ethyl vinyl acetate (EVA) or polypropylene or low density polyethylene.

9. Connecting member (1) according to one of claims 1 to 8, **characterised in that** the second piece (5) extends beyond the external contours of the first piece (4) by less than 1 mm, preferably by less than 0.5 mm.

10. Connecting member (1) according to one of claims 1 to 9, **characterised in that** the first piece (4) has a hardness between 0shA and 40shA, more particularly between 0shA and 20shA, and particularly 3shA.

11. Connecting member (1) according to one of claims 1 to 10, **characterised in that** the second piece(s) (5) have a hardness between 60shD and 90shD, more particularly between 70shD and 80shD, and particularly 76shD.

12. Connecting member (1) according to one of claims 2 to 11, **characterised in that** the third piece (15) has a hardness between 60shA and 100shA, more particularly between 80s hA and 100shA, and particularly 95shA, or between 60shD and 90shD, more particularly between 70shD and 80shD, and particularly 76shD.

13. Connecting member (1) according to one of claims 2 to 12, **characterised in that** spacer elements (25) consisting of rigid material are arranged between the second (5) and third (15) pieces.

14. Connecting member (1) according to claim 13, **characterised in that** the spacer elements (25) are integral with the second (5) and/or the third (15) piece.

15. Connector for an ostomy device, of the type comprising
a first connecting member (1) according to anyone of claims 1 to 14, mounted on a support (2) intended to be affixed to the skin of a user and
a second connecting member (8) mounted on a pouch (9) forming a receptacle, the second member (8) surrounding an opening (3) in the pouch (9),
the first member (1) having an external contour in a plane essentially parallel to the plane of the opening (3) in the support (2), and
the internal contour of the second member (8) in a plane essentially parallel to the plane of the opening (3) in the pouch (9) being essentially complementary but slightly larger than the external contour of the first member (1),
and the second member (8) comprising on its internal side (10) an elastically deforming lip (11) the free end of which is oriented towards the pouch (3), said lip (11) reducing the internal dimensions of the second member (8) to just below the external dimensions of the first member (1).

16. Connector for an ostomy device according to claim 15, wherein the lip (11) of said second member (8) is made from low density polyethylene.

17. Connector for an ostomy device according to claim 15 or claim 16, wherein the lip (11) of said second member (8) has a hardness between 20shD and 100shD, more particularly between 40shD and 60shD, and in particular 50shD.

## Patentansprüche

1. Verbindungselement (1) einer Ostomievorrichtung,
wobei das Element (1) auf einen Träger (2) montiert ist, der dazu beabsichtigt ist, auf die Haut eines Patienten fixiert zu sein und eine Öffnung (3) in dem Träger (2) zu umgeben, wobei das Element (1) einen ersten Teil (4) umfasst, der aus einem flexiblen Material besteht und mit zwei im Wesentlichen parallelen Seiten (6, 7), von denen die erste (6) an den Träger (2) fixiert ist, und mindestens einen zweiten Teil (5), der aus einem starren Material besteht, der auf die zweite Seite (7) des ersten Teils (4) fixiert ist und sich mindestens teilweise über die externen Umrisse des ersten Teils (4) hinaus erstreckt, **dadurch gekennzeichnet, dass** die Dicke des zweiten Teils (5) geringer als die Dicke des ersten Teils (4) ist.

2. Verbindungselement (1) nach Anspruch 1, **dadurch gekennzeichnet, dass** das Element (1) einen dritten Teil (15) umfasst, der aus einem starren Material besteht und zwischen dem ersten Teil (4) und dem Träger (2) angeordnet ist, wobei sich der dritte Teil (15) mindestens teilweise über die externen Umrisse des ersten Teils hinaus erstreckt.

3. Verbindungselement (1) nach Anspruch 1 oder 2, **dadurch gekennzeichnet, dass** der erste Teil (4) ringförmig ist.

4. Verbindungselement (1) nach Anspruch 3, **dadurch gekennzeichnet, dass** der zweite Teil (5) und/oder der dritte Teil (15) im Wesentlichen ringförmig sind, wobei ein äußerer Radius mindestens teilweise geringfügig größer als der Radius des ersten Teils (4) ist.

5. Verbindungselement (1) nach einem der Ansprüche 2 bis 4, **dadurch gekennzeichnet, dass** der dritte Teil (15) an den Träger entlang seines inneren Umfangs (16) fixiert ist, wobei der äußere Umfang (17) nicht an den Träger (2) fixiert ist.

6. Verbindungselement (1) nach einem der Ansprüche 1 bis 5, **dadurch gekennzeichnet, dass** der erste Teil (4) aus einem TPE (Thermoplastischem Elastomer), insbesondere StyrolButadien-Styrol (SBS), Styrol-Ethylen-Butadien-Styrol (SEBS), oder Ethylen-Vinylacetat (EVA) oder aus einem TPU (Thermoplastischen Polyurethan), aus einem Silikon oder aus einem Gummi hergestellt ist.

7. Verbindungselement (1) nach einem der Ansprüche 1 bis 6, **dadurch gekennzeichnet, dass** der zweite Teil (5) aus Polypropylen hergestellt ist.

8. Verbindungselement (1) nach einem der Ansprüche 2 bis 7, **dadurch gekennzeichnet, dass** der dritte Teil (15) aus einem TPE (Thermoplastischem Elastomer), insbesondere aus Ethylen-Vinylacetat (EVA) oder Polypropylen oder einem Polyethylen niedriger Dichte hergestellt ist.

9. Verbindungselement (1) nach einem der Ansprüche 1 bis 8, **dadurch gekennzeichnet, dass** sich der zweite Teil (5) über die externen Umrisse des ersten Teils (4) um weniger als 1 mm, vorzugsweise weniger als 0,5 mm hinaus erstreckt.

10. Verbindungselement (1) nach einem der Ansprüche 1 bis 9, **dadurch gekennzeichnet, dass** der erste Teil (4) eine Härte zwischen 0shA und 40shA, insbesondere zwischen 0shA und 20shA und besonders 3shA aufweist.

11. Verbindungselement (1) nach einem der Ansprüche 1 bis 10, **dadurch gekennzeichnet, dass** der/die zweite(n) Teil(e) (5) eine Härte zwischen 60shD und 90shD, insbesondere zwischen 70shD und 80shD und besonders 76shD aufweisen.

12. Verbindungselement (1) nach einem der Ansprüche 2 bis 11, **dadurch gekennzeichnet, dass** der dritte Teil (15) eine Härte zwischen 60shA und 100shA, insbesondere zwischen 80shA und 100shA und insbesondere 95shA oder zwischen 60shD and 90shD, insbesondere zwischen 70shD and 80shD und besonders 76shD aufweisen.

13. Verbindungselement (1) nach einem der Ansprüche 2 bis 12, **dadurch gekennzeichnet, dass** Abstandhalterelemente (25), die aus starrem Material bestehen, zwischen dem zweiten (5) und dritten (15) Teil angeordnet sind.

14. Verbindungselement (1) nach Anspruch 13, **dadurch gekennzeichnet, dass** die Abstandhalterelemente (25) einstückig mit dem zweiten (5) und/oder dem dritten (15) Teil sind.

15. Verbinder für eine Ostomievorrichtung, umfassend
ein erstes Verbindungelement (1) nach einem der Ansprüche 1 - 14, das auf einen Träger (2) montiert ist, das dazu beabsichtigt ist, auf die Haut eines Benutzers fixiert zu sein, und ein zweites Verbindungselement (8), das auf einen Beutel (9) montiert ist, der ein Gefäß bildet, wobei das zweite Element (8) eine Öffnung (3) im Beutel (9) umgibt, wobei das erste Element (1) einen äußeren Umriss in einer Ebene aufweist, die im Wesentlichen parallel zur Ebene der Öffnung (3) im Träger (2) ist, und wobei der innere Umriss des zweiten Elements (8) in einer Ebene, die im Wesentlichen parallel zur Ebene der Öffnung (3) im Beutel (9) ist, im Wesentlichen komplementär, jedoch geringfügig größer als der äußere Umfang des ersten Elements (1) ist, und wobei das zweite Element (8) an seiner inneren Seite (10) eine sich elastisch verformende Lippe (11) umfasst, deren freies Ende hin zum Beutel (3) ausgerichtet ist, wobei die Lippe (11) die inneren Abmessungen des zweiten Elements (8) auf gerade unter die externen Abmessungen des ersten Elements (1) reduziert.

16. Verbinder für eine Ostomievorrichtung nach Anspruch 15, wobei die Lippe (11) des zweiten Elements (8) aus Polyethylen niedriger Dichte hergestellt ist.

17. Verbinder für eine Ostomievorrichtung nach Anspruch 15 oder Anspruch 16, wobei die Lippe (11) des zweiten Elements (8) eine Härte zwischen 20shD und 100shD, insbesondere zwischen 40shD und 60shD und insbesondere 50shD aufweist.

## Revendications

1. Elément connecteur (1) d'un dispositif de stomie,
l'élément (1) étant monté sur un support (2) destiné à être fixé à la peau d'un patient et entourant une ouverture (3) dans ledit support (2)
l'élément (1) comprenant
une première pièce (4) constituée d'un matériau souple et comportant deux faces essentiellement parallèles (6, 7) dont la première (6) est fixée au support (2),
et au moins une deuxième pièce (5) constituée d'un matériau rigide,
étant fixée à la seconde face (7) de la première pièce (4)
et s'étendant au moins partiellement au-delà des contours externes de la première pièce (4),
**caractérisé en ce que**
l'épaisseur de la deuxième pièce (5) est inférieure à l'épaisseur de la première pièce (4).

2. Elément connecteur (1) selon la revendication 1, **caractérisé en ce que** l'élément (1) comprend une troisième pièce (15) constituée d'un matériau rigide et agencée entre la première pièce (4) et le support (2), la troisième pièce (15) s'étendant au moins partiellement au-delà des contours externes de la première pièce.

3. Elément connecteur (1) selon les revendications 1 ou 2, **caractérisé en ce que** la première pièce (4) est en forme d'anneau.

4. Elément connecteur (1) selon la revendication 3, **caractérisé en ce que** la deuxième pièce (5) et/ou la troisième pièce (15) sont essentiellement en forme d'anneau avec un rayon externe au moins en partie légèrement supérieur au rayon de la première pièce (4).

5. Elément connecteur (1) selon l'une des revendications 2 à 4, **caractérisé en ce que** la troisième pièce (15) est fixée au support le long de sa circonférence interne (16), la circonférence externe (17) n'étant pas fixée au support (2).

6. Elément connecteur (1) selon l'une des revendications 1 à 5, **caractérisé en ce que** la première pièce (4) est réalisée à partir d'un TPE (élastomère thermoplastique), en particulier dans du styrène butadiène styrène (SBS), du styrène éthylène butadiène styrène (SEBS), ou de l'éthyl-vinyl-acétate (EVA), ou à partir d'un TPU (polyuréthane thermoplastique), à partir d'une silicone ou à partir d'un caoutchouc.

7. Elément connecteur (1) selon l'une des revendications 1 à 6, **caractérisé en ce que** la deuxième pièce (5) est réalisée dans du polypropylène.

8. Elément connecteur (1) selon l'une des revendications 2 à 7, **caractérisé en ce que** la troisième pièce (15) est réalisée à partir d'un TPE (élastomère thermoplastique), en particulier dans de l'éthyl-vinyl-acétate (EVA) ou du polypropylène ou du polyéthylène basse densité.

9. Elément connecteur (1) selon l'une des revendications 1 à 8, **caractérisé en ce que** la deuxième pièce (5) s'étend au-delà des contours externes de la première pièce (4) de moins de 1 mm, de préférence de moins de 0,5 mm.

10. Elément connecteur (1) selon l'une des revendications 1 à 9, **caractérisé en ce que** la première pièce (4) a une dureté comprise entre 0 shA et 40 shA, plus particulièrement comprise entre 0 shA et 20 shA, et en particulier égale à 3 shA.

11. Elément connecteur (1) selon l'une des revendications 1 à 10, **caractérisé en ce que** la ou les deuxièmes pièces (5) ont une dureté comprise entre 60 shD et 90 shD, plus particulièrement comprise entre 70 shD et 80 shD, et en particulier égale à 76 shD.

12. Elément connecteur (1) selon l'une des revendications 2 à 11, **caractérisé en ce que** la troisième pièce (15) a une dureté comprise entre 60 shA et 100 shA, plus particulièrement comprise entre 80 shA et 100 shA, et en particulier égale à 95 shA, ou comprise entre 60 shD et 90 shD, plus particulièrement comprise entre 70 shD et 80 shD, et en particulier égale à 76 shD.

13. Elément connecteur (1) selon l'une des revendications 2 à 12, **caractérisé en ce que** des éléments écarteurs (25) constitués d'un matériau rigide sont agencés entre la deuxième (5) et la troisième pièce (15).

14. Elément connecteur (1) selon la revendication 13, **caractérisé en ce que** les éléments écarteurs (25) sont solidaires de la deuxième (5) et/ou de la troisième pièce (15).

15. Connecteur pour dispositif de stomie, du type comprenant
un premier élément connecteur (1) selon l'une quelconque des revendications 1 à 14, monté sur un support (2) destiné à être fixé à la peau d'un utilisateur et
un second élément connecteur (8) monté sur une poche (9) formant un réceptacle,
le second élément (8) entourant une ouverture (3) dans la poche (9),
le premier élément (1) ayant un contour externe situé dans un plan essentiellement parallèle au plan de l'ouverture (3) dans le support (2), et
le contour interne du second élément (8) dans un plan essentiellement parallèle au plan de l'ouverture (3) dans la poche (9) étant essentiellement complémentaire du contour externe du premier élément (1) mais légèrement plus grand que celui-ci,
et le second élément (8) comprenant sur son côté interne (10) une lèvre se déformant élastiquement (11) dont l'extrémité libre est orientée vers la poche (3), ladite lèvre(11) réduisant les dimensions internes du second élément (8) pour les rendre tout juste inférieures aux dimensions externes du premier élément (1).

16. Connecteur pour dispositif de stomie selon la revendication 15, dans lequel la lèvre (11) dudit second élément (8) est réalisée à partir de polyéthylène basse densité.

17. Connecteur pour dispositif de stomie selon la revendication 15 ou la revendication 16, dans lequel la lèvre (11) dudit second élément (8) a une dureté comprise entre 20 shD et 100 shD, plus particulièrement comprise entre 40 shD et 60 shD, et en particulier égale à 50 shD.
